Europäisches Patentamt

European Patent Office

Office européen des brevets

(11) Veröffentlichungsnummer: **0 077 054 B1**

(12) **EUROPÄISCHE PATENTSCHRIFT**

(45) Veröffentlichungstag der Patentschrift:
16.08.89

(21) Anmeldenummer: 82109349.9

(22) Anmeldetag: 08.10.82

(51) Int. Cl.⁴: **A 61 B 5/00**, A 61 B 5/04

(54) Messwertaufnehmer zur gleichzeitigen Messung verschiedener physiologischer Grössen.

(30) Priorität: 13.10.81 DE 3140673

(43) Veröffentlichungstag der Anmeldung:
20.04.83 Patentblatt 83/16

(45) Bekanntmachung des Hinweises auf die Patenterteilung:
16.08.89 Patentblatt 89/33

(84) Benannte Vertragsstaaten:
AT BE CH DE FR GB IT LI NL SE

(56) Entgegenhaltungen:
NL-A- 6 905 744

(73) Patentinhaber: Jensen, Arne, Dr., Am Hannes 4,
D-6301 Grossen-Linden Leihgestern (DE)

(72) Erfinder: Jensen, Arne, Dr., Am Hannes 4,
D-6301 Grossen-Linden Leihgestern (DE)

(74) Vertreter: WILHELMS, KILIAN & PARTNER
Patentanwälte, Eduard-Schmid-Strasse 2,
D-8000 München 90 (DE)

## Beschreibung

Die Erfindung betrifft einen Meßwertnehmer zur gleichzeitigen Messung verschiedener physiologischer Größen gemäß dem Oberbegriff des Patentanspruchs 1.

Meßwertaufnehmer, welche Meßkörper zur gleichzeitigen Messung verschiedener physiologischer Größen enthalten, sind in der Medizin immer dann von Wichtigkeit, wenn die Applikation einer großen Anzahl von Meßkörpern aus Zeit- oder Platzgründen oder im Hinblick auf eine Belastung des Patienten eine Erschwernis darstellt. Ein typisches Beispiel hierfür ist das Problem der Anbringung von Meßkörpern am Kopf oder Steiß des noch im Mutterleib befindlichen Fetus unter der Geburt.

Bei einem bekannten Meßwertaufnehmer der eingangs genannten Art (DE-A1-2 930 663) sind ein Aufnehmer zur polarographischen Messung des Sauerstoffpartialdruckes und ein Aufnehmer zur Messung von Herzaktionspotentialen (Elektrokardiogramm (EKG)) in einem Meßkopf miteinander kombiniert. Die EKG-Elektroden liegen bei diesem bekannten Meßwertaufnehmer alle in seiner Applikationsfläche, und zwar auf der auf dem Meßkörper angebrachten Mutter. In Anbetracht der notwendigerweise geringen Größe des Meßkopfes und damit seiner Applikationsfläche ist an dieser Anordnung der Elektroden nachteilig, daß wegen ihrer dadurch bedingten großen räumlichen Nähe die Gefahr eines Kurzschlusses zwischen den Elektroden erheblich ist. Außerdem sind die zu messenden Potentialdifferenzen gering und deshalb nur schlecht von Störpotentialen zu separieren. Bei dem bekannten Meßwertaufnehmer führt das Zuleitungskabel zu den EKG-Elektroden außerdem zu der Mutter.

Aus Medical instrumentation, Application and design; J. Webster, Houghton Mifflin Comp. Boston, 1978, Seiten 252–253, ist ein Meßkopf für die Feststellung fetaler Elektrokardiogramme bekannt, bei welchem sich die Referenzelektrode auf dem Rücken, d.h. auf der von der Applikationsfläche abgekehrten Seite des Meßkopfes, befindet.

Ein erfindungsgemäßer Meßwertaufnehmer, wie er im Patentanspruch 1 gekennzeichnet ist, schafft zu den oben erwähnten Nachteilen Abhilfe.

Eine bevorzugte Ausführungsform der Erfindung wird im folgenden in Verbindung mit der beigefügten Zeichnung beschrieben. Auf dieser zeigt

Fig. 1 einen Meßkopf gemäß dieser Ausführungsform in einem axialen Schnitt,

Fig. 2 in einer teilgeschnittenen Seitenansicht einen Meßkopf entsprechend Fig. 1 in einer Gestaltung, bei welcher die zweite Elektrode als Druckknopfstecker ausgebildet ist, und

Fig. 3 einen Klebeelektrodenkörper mit Druckknopfstecker in Seitenansicht.

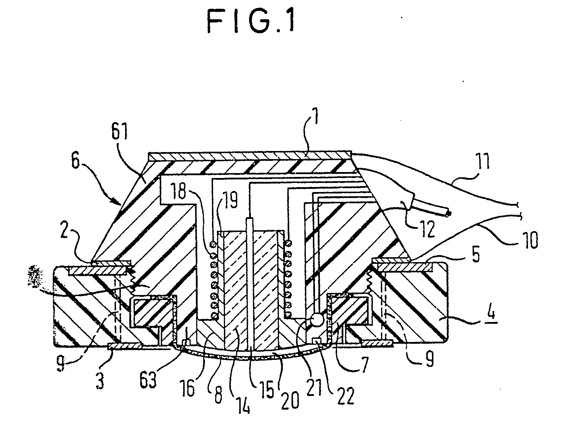

Fig. 1 zeigt einen Meßkopf zur gleichzeitigen Messung des transcutanen Sauerstoffpartialdrucks und der Herzaktionsspannung (Elektrokardiogramm (EKG)) bei noch im Uterus befindlichen Fetus unter der Geburt. Die Messung des transcutanen Sauerstoffpartialdrucks erfolgt bei dem dargestellten Meßkopf polarographisch.

Der Meßkopf weist einen Meßkopfkörper 6 aus Kunststoff auf, der geometrisch ein im wesentlichen axialsymmetrischer gestufter Aufbau mit einem oberen kegelstumpfförmigen Abschnitt 61, einem darunter anschließenden mit einem Gewinde versehenen zylindrischen Abschnitt 62 und einem wiederum darunter anschließenden ebenfalls zylindrischen Abschnitt 63 kleineren Durchmessers ist. Der zylindrische Abschnitt 63 kleineren Durchmessers ist von einem stufenförmigen Ring 7, ebenfalls aus Kunststoff, umgeben, dessen Innendurchmesser im wesentlichen dem Durchmesser des zylindrischen Abschnitts 63 kleineren Durchmessers entspricht und der rückseitig gegen den zylindrischen Abschnitt 62 größeren Durchmessers zur Anlage kommt. Im Spalt zwischen den zyindrischen Abschnitten 62 und 63 einerseits und dem Ring 7 andererseits ist eine sauerstoffdurchlässige Membran 8, etwa aus Polytetrafluoräthylen (PTFE), eingespannt, die sich vor der Stirnseite des zylindrischen Abschnitts 63 erstreckt und bei Anbringung des Meßkopfes etwa am Kopf des Feten mit diesem in Berührung kommt. Hinter der Membran 8 liegt axial eine aus einem in einen zylindrischen Glaskörper 14 eingeschmolzenen stirnseitig aber freien Draht, vorzugsweise aus Platin, bestehende Reduktionselektrode 15, an der durch die Membran 8 diffundierte Sauerstoffmoleküle reduziert werden, wobei der gemessene Reduktionsstrom ein Maß für den Sauerstoffpartialdruck in dem Gewebe (z.B. der Haut) ist, an dem der Meßkopf mit seiner Membran 8 angelegt ist. Die Reduktionselektrode ist koaxial von einer ringförmigen Referenzelektrode 16, vorzugsweise aus Silber, umgeben. Die Referenzelektrode wird durch eine Heizwicklung 18 beheizt, die um einen hohlzylindrischen Fortsatz 19 der Referenzelektrode 16 gelegt ist und bewirken soll, die Haut unter der Meßelektrode auf einer konstanten, erhöhten Temperatur zu halten, um eine möglichst gute Durchblutung und damit richtige Messung des Sauerstoffpartialdrucks zu gewährleisten. Zur Messung der Temperatur ist ein, z.B. durch einen Thermistor gebildeter, Temperaturfühler 21 vorgesehen. Zwischen der Membran 8 und der Stirnfläche von Reduktionselektrode 15, Glaskörper 14 und Referenzelektrode 16 befindet sich der für die polarographische Messung notwendige Elektrolyt 20, zu dessen Aufnahme auch eine Ringnut 21 in der Stirnseite des Abschnittes 63 vorgesehen ist. Der Glaskörper 14, in dem die Reduktionselektrode 15 eingeschmolzen ist, und die Referenzelektrode 16 sind leicht kalottenförmig ausgebildet, um der Membran 8 in ihrer Berührfläche etwa zur Haut einen leicht balligen Verlauf zu geben.

Auf das Gewinde des zylindrischen Abschnitts 62 ist eine Mutter 4 aus Kunststoff geschraubt, die an ihrem Innendurchmesser so gestuft ist, daß beim Aufschrauben der stufenförmige Ring 7 gegen den zylindrischen Abschnitt 62 gedrückt und die Membran 8 dadurch eingespannt wird. Die membranseitige Stirnfläche der Mutter 4 liegt mit der Membran 8 im wesentlichen in einer Ebene und enthält etwas aus der Ebene hervorspringend eingelassen einen Metallring 3, der zusammen mit der Membran 8 an der Haut zur Anlage kommt. Dieser Metallring 3 bildet die eine von zwei Elektroden zur Messung der Herzaktionsspannung, wobei die andere Elektrode außerhalb der Applikationsfläche als Metallplatte 1 auf der rückwärtigen, d.h. der der Membran abgekehrten Seite des Meßkopfes liegt. Diese abgekehrte Seite ist die kleine Stirnfläche des kegelstumpfförmigen Abschnitts 61. Der Metallring 3 steht über in axialer Richtung durch die Kunststoffmutter 4 verlaufende Schraubbolzen bzw. Stifte 9 mit einem weiteren Metallring 5 in Verbindung, der auf der der Applikationsfläche abgekehrten Seite der Mutter 4 eingelasser oder aufgesetzt ist. Bei aufgeschraubter Mutter 4 liegt der Metallring 5 gegen einen weiteren Metallring 2 an, der auf der radialen Absatzfläche zwischen kegelstumpfförmigem Abschnitt 61 und zylindrischem Abschnitt 62 angebracht ist. An diesem Metallring 2 ist die eine (10) von zwei Zuleitungen 10, 11 angeschlossen, die zu einem Elektrokardiogrammgerät führen. Die andere Zuleitung 11 ist an der Platte 1 angeschlossen. Mit diesem Aufbau ist erreicht, daß die Mutter 4 trotz elektrischer Verbindung des Metallrings 3 mit der Zuleitung 10 im aufgeschraubten Zustand abgenommen werden kann, ohne an einer Zuleitung zu hängen, daß also beide Zuleitungen zum Meßkopfkörper 6 führen. Am Meßkopfkörper 6 geht darüber hinaus auch das Kabel 12 für die Messung des Sauerstoffpartialdruckes weg.

Der Durchmesser des Meßkopfes beträgt außen an der Mutter 4 gemessen ca. 20 mm, seine Höhe und damit der Abstand der durch Metallplatte 1 und Metallring 3 gegeben Elektroden ca. 10 mm.

Zur Messung wird der Meßkopf etwa an den kindlichen Kopf bzw. Steiß geklebt. Der in der Schraubmutter 4 eingelassene Metallring 3 gelangt dabei zusammen mit der Membran in Kontakt zur kindlichen Haut. Die Klebung erfolgt dabei mittels einer Klebepaste so, daß diese auch den Metallring 3 umgibt und damit einen Zutritt der Fruchtwassernässe zu diesem verhindert. Wie bereits erwähnt, steht der am Meßkopfkörper 6 befindliche Metallring 2 über den Metallring 5 und die Schraubbolzen bzw. Stifte 9 in leitender Verbindung zu dem fetalseits liegenden eingelassenen Metallring 3 in der Haftfläche. Da durch die natürliche Nässe des Feten unter der Geburt eine Leitverbindung vom fetalen Körper zur Elektrode 1, wegen der Klebung nicht aber zum Metallring 3, hergestellt wird und auch unter Mitberücksichtigung des Fruchtwassers ein ausreichender Abstand zwischen den effektiven Elektroden verbleibt, ist mit dieser Anordnung die Registrierung des fetalen Elektrokardiogramms und über eine R-Zacken-Analyse eine Schlag-zu-Schlag-Herzfrequenzregistrierung des Feten möglich, neben der Messung des transcutanen Sauerstoffpartialdruckes $pO_2$ und einer bislang noch nicht erwähnten Hautdurchblutungsmessung, die auf der Messung des elektrischen Stromes beruht, der zur Erzeugung der erhöhten Hauttemperatur mittels der Silberheizelektrode notwendig ist; je besser die Haut durchblutet ist, desto größer ist die Wärmeabfuhr durch das Blut und damit der erforderliche Heizstrom.

Die beiden vom Meßkopf weggehenden Elektrokardiogramm-Zuleitungen 10 und 11 werden in den Steckkontakt handelsüblicher Kardiotokographen geklemmt, während das Zuleitungskabel 12, das die Anschlußleitungen für die Elektroden 15 und 16, die Heizwicklung 18 und den Temperaturfühler 21 enthält, an eine entsprechende signalverarbeitende Elektronik angeschlossen wird.

Mit dem beschriebenen Aufbau kann mit einem einzigen Meßkopf gleichzeitig die Herzfrequenzregistrierung, die Hautdurchblutungsregistrierung und die Registrierung des transcutanen Sauerstoffpartialdruckes zur Diagnose des fetalen Kreislaufschocksyndroms durchgeführt werden. Die Anbringung der durch die Metallplatte 1 gebildeten einen Elektrode auf der der Berührseite abgekehrten Seite des Meßkopfes sorgt dabei ohne zusätzlichen Platzbedarf für einen räumlichen Abstand der beiden wirksamen Elektrokardiogrammelektroden, der gut meßbare Potentialdifferenzen liefert.

Der Meskopf wurde unter der Geburt getestet, wobei die fetale Herzfrequenz (über Kardiotokograph von HEWLETT PACKARD 8030), die Wehentätigkeit (direkt über Kardiotokograph von HEWLETT PACKARD 8030), die relative lokale Hautperfusion (über Oxymonitor (HELLIGE)) und der transcutane fetale $pO_2$ (über Oxymonitor (HELLIGE)) gemessen wurden. Alle Signale wurden einwandfrei bis zur Geburt registriert.

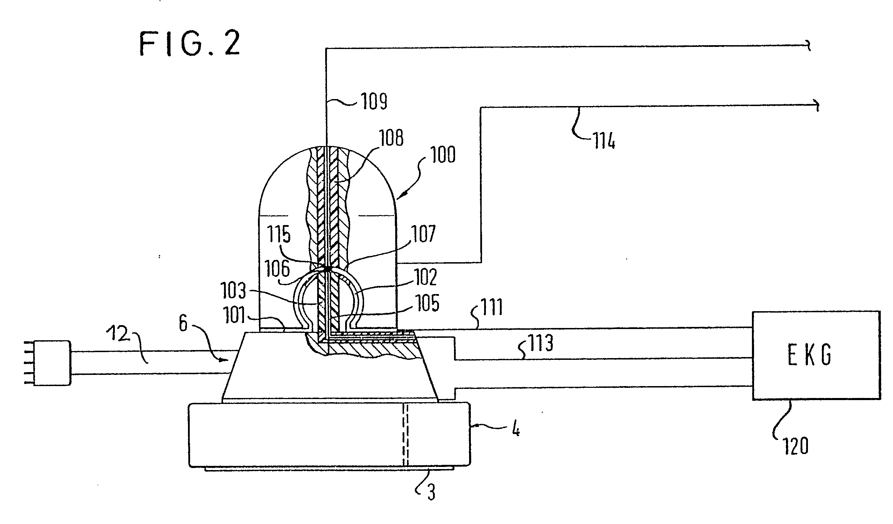

Fig. 2 zeigt einen Meßwertaufnehmer, der vor allem eine von vorhandener Nässe bzw. Elektrolytcreme unabhängige Messung gestattet und außerdem zu einer Impedanzmessung im Hinblick auf eine Messung der Atemfrequenz geeignet ist.

Der Meßkopf ist hinsichtlich des Meßkopfkörpers 6 und der damit zusammenwirkenden Mutter 4 gleich aufgebaut wie der Meßkopf der Fig. 1. Auch die in seinem Inneren befindliche Einrichtung zur polarographischen Messung ist hier in der gleichen Weise vorhanden wie dort.

Unterschiede sind hinsichtlich des folgenden Aufbaus vorhanden. Die Metallplatte 1 der Fig. 1 ist durch eine Metallplatte 101 ersetzt, die in der Mitte das männliche Teil 102 eines Druckknopfsteckers 100 enthält. Dieses Teil 102 weist eine axiale Bohrung auf, die mit Kunststoff 103 gefüllt ist, und in der durch diesen gegenüber dem übrigen Kontaktkörper des Teils 102 isoliert eine Leitung 105 geführt ist, die sich, ebenfalls durch Kunststoff auch gegenüber der Metallplatte 101 isoliert im Kopfteil fortsetzt und als Leitung 113 zu einem Elektrokardiogramm-Gerät 120 weiterverläuft. Die Platte 101 selbst steht über eine Leitung 111 mit dem Elektrokardiogramm-Gerät in Verbindung. Die durch das Teil 102 des Druckknopfsteckers verlaufende Leitung 105 endet in einem Kontaktpunkt 106, der in der Achse des Teils 102 liegt.

Beim Gegenstück des Druckknopfsteckers liegt das weibliche Teil 107 bei geschlossener Verbindung in Berührung mit dem männlichen Teil 102.

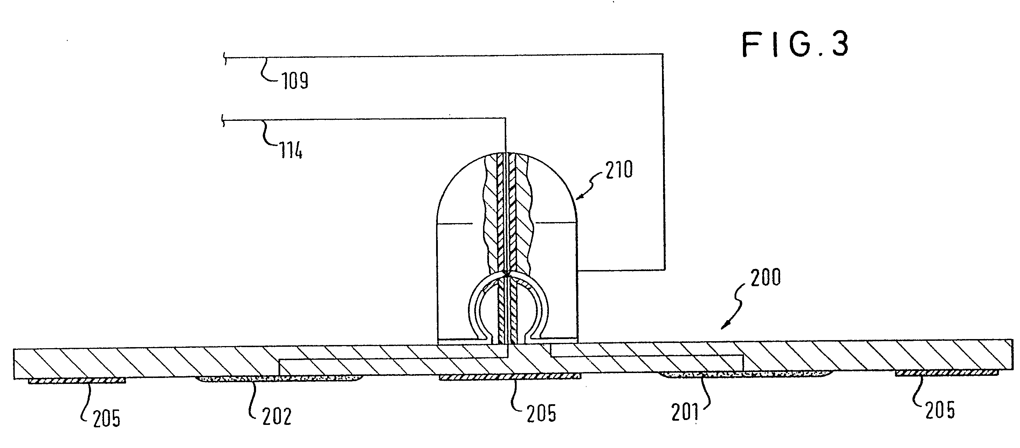

Auch das weibliche Teil 107 ist axial durchbohrt, wobei in der Bohrung durch Kunststoff 108 gegenüber dem Teil 107 isoliert eine Leitung 109 geführt ist, über die eine erste Elektrode 201 eines in Fig. 3 gezeigten Klebeelektrodenkörpers 200 angeschlossen ist. Eine mit einer zweiten Elektrode 202 des Klebeelektrodenkörpers in Verbindung stehende Leitung 114 ist an den Körper des weiblichen Teils 107 angeschlossen.

Ebenso wie die Leitung 105 in einem Kontaktpunkt 106 endet die Leitung 109 in der Achse des weiblichen Teils 107 in einem Kontaktpunkt 115. Bei zusammengefügter Steckverbindung kommen die Kontaktpunkte 106 und 115 miteinander in Berührung und stellen so die Verbindung zwischen den Leitungen 105 und 109 her.

Der in Fig. 3 gezeigte Klebeelektrodenkörper 200 ist eine elliptische oder kreisförmige Scheibe, auf der eine erste Elektrode 201 und eine zweite Elektrode 202 als kreisförmige Scheiben vorgesehen sind, wobei eine Klebefolie 205 die Elektroden umgibt.

Zum Anschluß der Elektroden 201 und 202 an die Leitungen 109 und 114 ist eine einesteils an diesen Leitungen und anderenteils am Klebeelektrodenkörper 200 angebrachte Druckknopfsteckverbindung 210 vorgesehen, die einschließlich der axialen Durchführungen sowie der Anschlußpunkte völlig analog zur meßkopfseitigen Druckknopfverbindung 100 aufgebaut ist.

Bei dieser Ausführungsform des Meßwertmitnehmers werden die Herzaktionspotentiale vom Körper des Patienten über die Elektroden 201 und 202 sowie die durch den Metallring 3 gegebene Elektrode abgenommen.

Diese Ausführungsform von Meßwertaufnehmern gestattet eine gleichzeitige Messung eines Gaspartialdruckes und von Herzaktionsströmen mit drei Elektroden mit insgesamt nur zwei Meßkörpern. Für die gleiche Messung waren ursprünglich vier Meßkörper, nämlich ein Meßkopf für die Messung des Gaspartialdruckes und drei Einzelelektroden für die Messung der Herzaktionspotentiale, notwendig.

Da sich bei entsprechender Anbringung der Elektrodenkörper und der Meßkopf beim Heben und Senken des Brustkorbes relativ zueinander bewegen können, was über eine Impedanzmessung eine Messung der Atemfrequenz gestattet, ist dieser Ausführungsform gegenüber der ersten Ausführungsform ein weiterer Vorteil gegeben.

## Patentansprüche

1. Meßwertaufnehmer zur gleichzeitigen Messung verschiedener physiologischer Größen, mit einem Meßkopf, welcher einen Aufnehmer zur Messung einer ersten physiologischen Größe und einen mindestens eine mit einer Signalverarbeitungselektronik verbindbare erste und zweite Elektrode (1, 3) aufweisenden Aufnehmer zur Messung einer bioelektrischen Größe als einer weiteren physiologischen Größe enthält und eine Applikationsfläche, mit der er am menschlichen oder tierischen Körper zur Anlage bringbar ist, aufweist, wobei die mindestens eine erste Elektrode (3) zusammen mit der Applikationsfläche mit dem menschlichen oder tierischen Körper in Berührung kommend am Meßkopf angeordnet ist, wobei der Meßkopf einen den Aufnehmer für die Messung der ersten physiologischen Größe enthaltenden Meßkopfkörper (6) und eine auf dem Meßkopfkörper (6) anbringbare Mutter (4), welche die mindestens eine erste Elektrode (3) enthält, aufweist, dadurch gekennzeichnet, daß die mindestens eine zweite Elektrode (1) außerhalb der Applikationsfläche des Meßkopfs auf dem Meßkopfkörper (6) angebracht ist und die mindestens eine erste Elektrode (3) mit einer auf der ihr abgekehrten Seite der Mutter (4) vorgesehenen ersten Metallplatte (5) in Verbindung steht, die bei auf dem Meßkopfkörper (6) angebrachter Mutter (4) mit einer am Meßkopfkörper (6) vorgesehenen zweiten Metallplatte (2) in Berührung steht, die an die Signalverarbeitungselektronik angeschlossen ist.

2. Meßwertaufnehmer nach Anspruch 1, dadurch gekennzeichnet, daß die erste Elektrode als ein mit der Mutter (4) koaxialer Metallring (3) vorgesehen ist.

3. Meßwertaufnehmer nach Anspruch 1 oder 2, dadurch gekennzeichnet, daß auf dem Meßkopf ein bipolarer Steckanschluß angebracht ist, dessen einer Pol durch Verbindung mit der zweiten Elektrode (1) mit der Signalverarbeitungselektronik verbindbar ist und dessen anderer Pol ebenfalls mit der Signalverarbeitungselektronik verbindbar ist.

4. Meßwertaufnehmer nach Anspruch 3, dadurch gekennzeichnet, daß der bipolare Steckanschluß durch einen Durchknopfsteckanschluß gebildet ist, wobei der Druckknopfkörper den einen Pol und ein demgegenüber isolierter axialer Kern den zweiten Pol bildet.

## Claims

1. Transducer for the simultaneous measurement of various physiological magnitudes, with a measuring head which contains a sensor for measuring a first physiological magnitude and, for measuring a bioelectrical value as a further physiological magnitude, a sensor comprising at least one first and second electrode (1, 3) adapted to be connected to an electronic signal processing unit, and also comprising an application surface by which it can be brought to bear on the human or animal body, the at least one first electrode (3) being disposed on the measuring head which comes in contact with the human or animal body, the measuring head having a measuring head body (6) containing the sensor for measuring the first physiological magnitude and a nut (4) adapted to be mounted on the measuring head body (6) and containing the at least one first electrode (3), characterised in that the at least one second electrode (1) is mounted on the measuring head body (6) outside the application surface of the measuring head and the at least one first electrode (3) is in communication with a first metal plate (5) provided on the side of the nut (4) which is remote from the said electrode and which, when the nut (4) is mounted on the measuring head body (6), comes in contact with a second metal plate (2) provided on the measuring head body (6), the said second metal plate being connected to the electronic signal processing unit.

2. Transducer according to Claim 1, characterised in that the first electrode is provided in the form of a metal ring (3) coaxial with the nut (4).

3. Transducer according to Claim 1 or 2, characterised in that there is on the measuring head a bipolar plug connection, of which one terminal can be connected to the electronic signal processing unit by being connected to the second electrode (1) while the other terminal can likewise be connected to the electronic signal processing unit.

4. Transducer according to Claim 3, characterised in that the bipolar plug connection consists of a push button plug connection, the push button body constituting one terminal while the axial core which is insulated in respect of it constitutes the second terminal.

**Revendications**

1. Capteur de valeurs mesurées pour la mesure simultanée de différentes grandeurs physiologiques, comportant une tête de mesure, qui contient un capteur pour la mesure d'une première grandeur physiologique et un capteur comprenant au moins une première et une seconde électrode (1, 3) pouvant être reliées à une électronique de traitement des signaux pour la mesure d'une grandeur bioélectrique telle qu'une autre grandeur physiologique, ledit capteur de valeurs mesurées comportant également une surface d'application au moyen de laquelle il peut être appliqué sur un corps humain ou animal, capteur dans lequel au moins ladite première électrode (3) ainsi que la surface d'application sont disposées sur la tête de mesure pour pouvoir venir en contact avec le corps humain ou animal, capteur dans lequel la tête de mesure comporte un corps (6) de tête de mesure contenant le capteur pour la mesure de la première grandeur physiologique et un écrou (4) pouvant être rapporté sur le corps (6) de tête de mesure, cet écrou contenant au moins ladite première électrode (3), caractérisé en ce qu'au moins ladite seconde électrode (1) est rapportée sur le corps (6) de tête de mesure à l'extérieur de la surface d'application de la tête de mesure, et au moins ladite première électrode (3) est reliée avec une première plaque de métal (5) prévue sur le côté opposé de l'écrou (4), et ladite plaque métallique (5) est en contact avec une seconde plaque métallique (2) prévue dans le corps (6) de la tête de mesure, cette seconde plaque métallique étant reliée à l'électronique de traitement des signaux.

2. Capteur selon la revendication 1, caractérisé en ce que la première électrode est réalisée sous la forme d'une bague métallique (3) coaxiale à l'écrou (4).

3. Capteur selon l'une des revendications 1 et 2, caractérisé en ce qu'une prise de courant embrochable bipolaire est montée sur la tête de mesure, et un pôle de cette prise peut être relié à l'électronique de traitement des signaux par liaison avec la seconde électrode (1), tandis que l'autre pôle de la prise peut être également relié à l'électronique de traitement des signaux.

4. Capteur selon la revendication 3, caractérisé en ce que la prise de courant embrochable bipolaire est formée par un bouton-poussoir, qui constitue un pôle et d'autre part un noyau axial isolé du second pôle.

# FIG.1

# FIG.2

EKG

EP 0 077 054 B1

FIG.3

109

114

210

200

205    202    205    201    205

EP 0 077 054 B1